# EUROPEAN PATENT APPLICATION

(11) **EP 3 912 548 A1**
(43) Date of publication of application: **24.11.2021**
(21) Application number: 21020271.9
(22) Date of filing: 21.05.2021
(51) Int. Cl.: A61B 5/00, A61B 5/256, A61B 5/291, A61B 5/297

(54) **WEARABLE ELECTROENCEPHALOGRAPHIC MONITORING DEVICE**

(30) Priority: 22.05.2020 US 202063028632 P; 05.07.2020 US 202063048144 P
(71) Applicant: Chiang, Hsin-Yin, 67000 Strasbourg (FR)
(72) Inventor: Chiang, Hsin-Yin, 67000 Strasbourg (FR)

(57) **Abstract**

The present application generally relates to devices for monitoring, detecting, and processing electroencephalogram (EEG) signals of the human brain. More specifically, the present invention discloses an instant and discrete EEG monitoring device in forms of an eyewear adapter and an earwear. The EEG monitoring device of an eyewear adapter can be applied on every eyewear, while that of an earwear can be worn directly on ear(s) preferably with skin adhesive integrated. The EEG monitoring device comprises a flexible printed circuit (FPC) with electrode attachment and one or more elastic material(s) overmolding. The overall device is flexible, slim, and size adjustable, facilitating EEG monitoring comfortably, inconspicuously, and continuously to capture the brain electricity of patients and users in real life.

## Description

### Field of the Invention

The present application generally relates to devices for monitoring, detecting, and processing electroencephalogram (EEG) signals of the human brain. More specifically, the present invention discloses an instant and discrete EEG monitoring device in the forms of an earwear and an eyewear adapter.

### Background

Generally speaking, EEG is a monitoring method that records the electrical activity of the brain. EEG measures the brainwaves noninvasively via electrodes/sensors placed on the scalp and helps to establish an accurate diagnosis of brain activity. In neurology, one of the common diagnostic applications of EEG is in diagnosing epilepsy. For patients with epilepsy, it is crucial, for medical professionals, to detect the unusual electrical activity in the brain when a seizure is triggered. When the patients do not experience a seizure the brain activity may remain normal. This means unless the patients experience a seizure during EEG recording, the doctor cannot diagnose the type of seizure in full confidence. Due to the unpredictable occurrence of seizures and the limited consulting duration per patient, e.g. a session of EEG in hospital, there is indeed an urgent need for a portable and inconspicuous EEG device that can be continuously worn by the patients throughout the day to overcome the current constraints of laboratory-based or hospital-based EEG tests.

Likewise, EEG monitoring also facilitates to optimize the efficiency of medical treatment of mental disorders, Parkinson's disease, and Alzheimer's disease. Several studies show that EEG signals can be used to determine the mental status, emotions, and moods of a user, and it has been applied to diagnose the mental disorders of patients. The detection of emotional profile via EEG signals is particularly important as it reflects the symptoms of mental disorders in the early stage and can be used to derive the patient's mood pattern (i.e. mood tracking), tracking the efficacy of the designated treatment accurately. In addition, the patient's emotional triggers can be found and further resolved with the professional's help to improve the life quality of the patient. At the moment, mood tracking is done manually by the patient to log at fixed time slots. It often lacks accuracy as it relies on memories to recall the moods throughout a day and is therefore adversely susceptible to recall bias. Furthermore, the act of recalling negative feelings may aggravate the mental status of the patient. The application of EEG monitoring can resolve these inconveniences by providing automatic mood tracking. To achieve mood tracking for a long duration throughout the day, having an invisible or inconspicuous EEG monitoring device is desirable for the field of mental health.

Alzheimer's disease (AD) is a neurodegenerative disorder that is characterized by cognitive deficits that result in the reduced capacity of patients in daily life and behavioral disturbances. EEG has been demonstrated as a reliable tool in the research of AD and its diagnosis as it contributes to the differential diagnosis and the prognosis of the disease progression. Additionally, such recordings of EEG can add important information related to drug effectiveness. Similarly, EEG has been proven to be necessary for efficiently managing Parkinson's disease. A portable and invisible or inconspicuous EEG device can facilitate the monitoring of these diseases without causing additional disturbances in daily life.

For all of these diseases and many more, effective monitoring of brain activities via EEG for a long duration is essential. This is because certain health information such as the occurrence of emotional triggers and other abnormalities of electrical activity in the brain does not only take place exclusively in hospitals, in laboratories, or in private, but anytime, anywhere. Various systems for monitoring EEG have been known for several years and with the general technological development, EEG monitoring systems, which may be worn continuously by a person to be monitored, have been devised. However, most of the existing prior art EEG devices have bulky and obvious structures that can be worn exclusively in laboratories or in private.

### Prior Art

In the existing prior art, several efforts have been made to provide EEG monitoring devices. In some of the devices one or more sensors integrated as a part of earwear, for example, US20190053766, KR101579364, and so on. In some other devices one or more electrodes integrated as a part of eyewear, for example, US9016857B2, US20160054569, CN103976733A, CN103690161A, and so on.

One issue with such systems is that the EEG monitoring devices described in the prior art are not size adjustable, and therefore may not be appropriate for all users. Another issue with such systems, particularly those of earwear, is that many of the EEG monitoring devices described in the prior art are bulky and thus impractical for wearing inconspicuously throughout the duration of a user's day.

Another issue with prior systems of EEG monitoring devices in a form of eyewear is that their sensors/electrodes are built in a specific piece of eyewear that just has one fixed structural configuration for all users. In such EEG systems embedded in eyewear, it is a fact that eyewear cannot be easily replaced by users due to the configured EEG monitoring device or sensors therein. The fixed structure of the known eyewear limits the adjustment to the individual user's head size. So these existing EEG monitoring devices fail in fitting on the user comfortably. Consequently, these systems have issues such as discomfort during the use and poor quality of EEG signals. Furthermore, eyewear nowadays is not only considered as a tool, but also a fashion accessory. Imposing EEG-embedded eyewear with fixed appearances can limit the user's acceptance and further degrade the user's engagement with EEG monitoring.

In order to overcome the aforementioned issues, the inventor herein proposes two types of EEG monitoring systems: an eyewear adapter and an earwear which conveniently and accurately place EEG electrodes in contact with skin on a user's skull. They are size-adjustable, flexible, and may be worn on or around a user's ear or used as an eyewear adapter inconspicuously or invisibly.

### Summary of the Invention

The proposed EEG monitoring device in two forms of an eyewear adapter or an earwear comprises: at least one EEG electrode and a main structure with a flexible printed circuit (FPC) which houses the EEG electrode(s), facilitating stable contact between the electrode (s) and a user's scalp.

The proposed EEG monitoring device is easily size-adjustable, and is able to collect resourceful electroencephalographic information of users. The application of EEG dry electrode(s) makes this proposed EEG monitoring device easy to use without extra preparation and suitable for all kinds of users. The dry electrode(s) may comprise a conductive polymer, non-skin-irritating metal, conductive ink, or conductive hydrogel.

The proposed EEG monitoring device has an invisible or inconspicuous design, assuring continuous EEG monitoring throughout daily life. The EEG monitoring device ensures convenient and reliable placement of EEG electrodes on preselected positions of the user's skull according the standard 10-10 system. The proposed EEG monitoring device ensures the user's maximum comfort and is capable of ensuring stable EEG signal acquisition.

A FPC may act as an internal framework of the EEG measuring device and comprises a resilient flexible material. The FPC may be made of but not limited to Polyethylene terephthalate (PET) and Polyimide (PI).

Some embodiments of the EEG monitoring device may further comprise one or more electrooculographic (EOG) electrodes, measuring the corneo-retinal standing potential that exists between the front and the back of the human eye, in addition to the EEG electrode(s). In those forms, the EEG monitoring device is also capable of measuring EOG signals for further information such as facial expression recognition.

The EEG monitoring device of an eyewear adapter can be attached or configured on any existing eyewear without changing the overall look of the eyewear. The removable design of the proposed eyewear adapter gives users the freedom to mount it over different eyewear to optimize the user's engagement with, and adherence to, EEG monitoring.

The EEG monitoring device of an eyewear adapter may comprise: a main structure in the shape of a sleeve and one or more EEG electrodes. The sleeve referred here is not limited to a tubular shape and may be partially tubular. The sleeve refers to a shape that can slide over or grip the intended temple to anchor the positions of electrode(s). The sleeve may have at least one opening which is sized and configured to slide over an intended temple of the eyewear therein with a distal end near the intended temple tip behind the ear. The sleeve may have opposite open ends or may have an open end and a distal end which is closed. The sleeve may be in a curved shape, curving downward along the intended temple tip(s).

The sleeve (the main structure) of an EEG eyewear adapter may comprise a FPC which may have a flexible strip-like form or sheetlike form. The main structure or the sleeve may hold the strip-like or sheetlike form parallel to the temple. The sleeve may be flexible so that it may slide over the intended temple easily and accommodate the curvature of the eyewear temple.

The sleeve may be semi-rigid with a pre-formed shape conforming to a temple. The sleeve may be flexible or stretchable to conform to the shape of the temple.

The sleeve may comprise one or more elastic materials, such as but not limited to thermoplastic polyurethane (TPU), silicone rubber, nitrile rubber, and other synthetic rubbers, overmolding over the FPC in the region without electrode attached and be flexible to adapt the user's skull. In other words, the FPC is covered by electrode(s) and by elastic material(s).

In some embodiments, the main structure, the sleeve, may comprises one or more electrodes located in the positions of T9/T10, correspond to the top contact point between the helix of ear and the user's skull, and A1/A2 of the 10-10 system, corresponding to mastoid of the user's skull.

The sleeve may also comprise a strip with a slider in the form of a ring, clasp, or connector, extending towards the hinged end of the intended temple. The slider is configured to fit or attach over the temple of the eyewear. The strip may be made of the same FPC of the sleeve and the slider may be made of one or more elastic materials such as but not limited to thermoplastic polyurethane (TPU), silicone rubber, nitrile rubber, and other synthetic rubbers. This combination of a strip and a slider is situated in the inner side of the eyewear temple. The sleeve may be fixed to an end of the strip to hold the end to the temple.

The strip houses one or more EEG electrodes and by moving the slider toward the sleeve, the strip forms a protruding structure, facilitating the contact of the EEG electrode(s) in the position of FT9/FT10 of the 10-10 system, corresponding to the temple area of human skull. An EOG electrode may also be mounted on the strip to be in contact with the position FT9/FT10.

By moving the slider toward or away from the sleeve, one can adjust the adequate pressure applied on the skull, ensuring satisfying signal quality of EEG measurement.

The user positions the EEG electrodes by simply sliding the ring toward either the hinged end of the temple or toward the opposite end where there is the main structure by using the fingers.

The EEG monitoring device in a form of eyewear adapter may comprise one or two flexible sleeve(s), one or two strips and sliders, the associated electrodes, and a processing unit which may be disposed within the main structure or externally with an electrical connection. The EEG and EOG electrodes may be dry electrodes made of or comprising conductive polymer, non-skin-irritating metal, conductive ink, and conductive hydrogel.

In another form the EEG monitoring device may be in the form of an earwear, comprising at least one EEG electrode, at least one ear hook, and a signal processing unit. The ear hook comprises a FPC, in a sheetlike form and coplanar with the ear hook, one or more electrodes attached on that FPC, and one or more elastic materials, such as thermoplastic polyurethane (TPU), silicone rubber, nitrile rubber, and other synthetic rubbers, overmolding over that FPC on the region without electrode(s). In other words, the FPC is covered by electrode(s) and by elastic material(s). The one or more EEG electrodes of the ear hook may be positioned at T9/T10 and A1/A2 of the 10-10 system when in use. The ear hook is placed between the helix of ear and the user's skull, parallel to the median sagittal plane.

This EEG monitoring device of earwear may comprise a flexible extension, extending from the ear hook toward the temple area of the user's skull. This flexible extension may host one or more EEG electrodes or EOG electrodes to be in contact with the positions of FT9 and FT10 of the 10-10 system. The extension is bendable and flexible, facilitating size adjustment to the individual users.

The EEG monitoring device of an earwear may comprise an adhesive to ensure contact with the user's head preferably in two regions. First, the adhesive is applied around the main structure, including the contour of the ear hook and the extension. The adhesive may be a pressure sensitive adhesive (PSA) or non-conductive biomimetic adhesive. Second, a skin adhesive is integrated surrounding or in the EEG or EOG electrode(s). The adhesive may be a pressure sensitive adhesive (PSA) or non-conductive biomimetic adhesive, or directly as adhesive electrode(s), using conductive PSA or biomimetic adhesive.

The EEG monitoring device in a form of earwear may comprise one or two ear hooks, one or two flexible extension, the associated electrodes, and a processing unit which may be disposed within the main structure or externally with an electrical connection. The EEG and EOG electrode may be dry electrodes made of or comprise conductive polymer, non-skin-irritating metal, conductive ink, and conductive hydrogel.

The EEG monitoring device in a form of an earwear is further disclosed in dependent claims.

The EEG monitoring device of both an eyewear adapter and an earwear comprises a signal processing unit that is located in the main structure, the sleeve or the ear hook, in a concealed manner. The processing unit may be located externally and electrically connected to one or more EEG or EOG electrode(s). The processing unit may be connected to the electrode(s) via the FPC and/or by an electrical cable. The processing unit may comprise at least one of an analog to digital converter, a power supply, a data processor, a transceiver/communication module, a memory, and a display. The memory may include a machine learning or deep learning algorithm stored therein.

The FPC with electrode attachment may act as an electrical circuit for the device. The FPC is preferably able to limit the overall cross-sectional dimension of the device by working as a substrate to house the sensors. In other devices that exist in the art, EEG sensors made of polymers are housed by a metallic substrate that is afterwards soldered into or mechanically in contact with the rest of an electrical connection. To attach the sensor on this metallic insert, the minimum thickness including the metallic substrate is around 3-6 mm due to the processing constraints and the sensor will be rigid due to the metallic substrate. By utilizing an FPC, the combined thickness of a sensor and an electrical connection of the present invention can be 1-2 mm or even lower. This allows the overall device to be slim, flexible, and inconspicuous. This fact is particularly beneficial to the proposed embodiment as a portable EEG measuring device as the lighter design can assure the maximum comfort and the inconspicuousness of the product when in use, further enhancing user engagement on brainwave detection.

The FPC overmolded or encapsulated by one or more elastic materials facilitates freedom of movement to the EEG monitoring device thereof in the direction toward or away from the user's head parallel to the frontal plane and prevent movement in the direction parallel to the sagittal plane of the user. The size and/or the shape of the EEG monitoring may be adjusted by a user by bending the one or more flexible printed circuits.

The EEG monitoring device may comprise at least four electrode positions among FT9, FT10, T9, T10, A1, and A2 of a 10-10 system when on both sides of the user's skull or at least two electrode positions among FT9, T9 and A1 or FT10, T10, and A2 when on single side of the user's skull.

The proposed locations of the EEG and/or EOG electrode(s) on the user's head are crucial to provide detailed information of electrical activities in the user's brain. The specific EEG and or EOG electrode locations described herein (FT9, FT10, T9, T10, A1, and A2) are the designated positions in the 10-10 EEG system and are able to form at least 10 channels, said 10 channels being FT9-FT10, FT9-T9, FT9-T10, FT9-A1, FT10-T9, FT10-T10, FT10-A1, T9-T10, T9-A1, and T10-A1. These channels are able to detect various brainwaves such as alpha, beta, and gamma waves as well as to provide the correlation levels for designated applications such as emotion recognition.

For example, the channel T9-T10 is able to provide the correlation of neutral-positive emotions via analyzing alpha wave, FT9-T9 for the correlation of negative-neutral emotions via both alpha and beta waves, and FT10-T10 for phase synchronization of negative-positive emotions via beta wave. Furthermore, these channels are capable of detecting different statuses of a user's brain as arousal and valence levels. FT9-A1 and FT10-A2 are able to provide EOG signals that can be used for facial expression recognition. For standard EEG acquisition, it is essential to have the reference points such as A1 and A2 to obtain local bio-potentials at individual EEG positions.

Further disclosure of the EEG monitoring device is provided in the claims.

These and other features and advantages of the present invention will become apparent from the detailed description below, in light of the accompanying drawings.

The invention will now be described, by way of example only, with reference to the accompanying figures in which:

### Brief Description of the Figures

FIG. 1 is a diagram that illustrates a 10-10 system internationally recognized and that allows EEG electrode placement to be standardized for effective EEG monitoring;
FIG. 2 is a diagram that illustrates a conventional 10-20 system;
FIG. 3 is a diagram that illustrates a bony region behind the ears for the placement of the reference electrode(s) for effective EEG monitoring;
FIG. 4 shows an eyewear adapter device mounted on eyewear, according to an embodiment of the present invention;
FIG. 5 shows an eyewear adapter device mounted on eyewear, according to another embodiment of the present invention;
FIGS. 6-7 show exemplary use case scenarios for eyewear embodying the eyewear adapter of FIG. 5;
FIG. 8 shows an eyewear adapter device mounted on eyewear, according to another embodiment of the present invention;
FIG. 9 shows an eyewear adapter device mounted on eyewear, according to another embodiment of the present invention;
FIG. 10 is an exploded view showing eyewear and an eyewear adapter according to another embodiment of the present invention;
FIGS. 11-12 show exemplary use case scenarios for eyewear embodying the eyewear adapter of FIG. 10;
FIG. 13 is an exploded view showing eyewear and an eyewear adapter according to yet another embodiment of the present invention;
FIG. 14 shows an eyewear adapter device mounted on eyewear, according to another embodiment of the present invention;
FIG. 15 shows an eyewear adapter device mounted on eyewear, according to another embodiment of the present invention;
FIG. 16 shows an exploded view showing eyewear and an eyewear adapter according to yet another embodiment of the present invention;
FIGS. 17A-17C show a general material organization of the proposed eyewear adapter;
FIGS. 17D-17E are diagrams that demonstrate the possible adjustment of the eyewear adapter device mounted on the eyewear, according to an embodiment of the present invention;
FIG. 18 illustrates an on-ear embodiment of the invention;
FIG. 19 illustrates an on=ear embodiment of the invention worn by a user;
FIG. 20 illustrates a close-up view of an on ear embodiment of the invention;
FIG. 21 illustrates the adjustability of the extension of the proposed earwear;
FIG. 22 illustrates the adjustability of the extension of the proposed earwear when worn by a user;
FIG. 23 is an exemplary block diagram that shows a processing unit used for processing the EEG signals acquired by EEG electrodes;
FIG. 24 is an exemplary environmental diagram that shows the communication between the processing unit of the EEG adapter mounted on the eyewear and the mobile communication device and/or medical facility; and
FIG. 25 illustrates orientation of the median sagittal plane (1460) and the frontal plane (1440) with respect to a user.

### Detailed Description of the Invention

Before describing the present invention in detail, it should be understood that the present invention utilizes a combination of components as a result of an instant and invisible or inconspicuous EEG monitoring device in the forms of an eyewear adapter or as an earwear.

The EEG monitoring device of the present invention will be described with reference to the accompanying drawings, which should be regarded as merely illustrative without restricting the scope and ambition of the present invention.

The proposed EEG monitoring device of an eyewear adapter facilitates EEG measurement for users or patients in a more continuous and simpler fashion. One can use the removable adapter upon eyewear to conduct EEG measurements invisibly and inconspicuously in daily life and receive the measurement result which can be further shared with medical professionals.

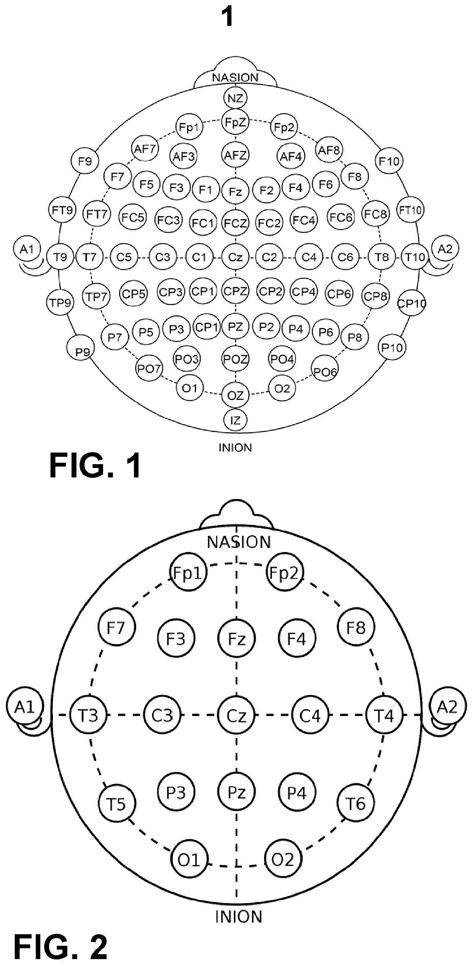

FIG. 1 is a diagram that illustrates a 10-10 system which is internationally recognized and ensures the placement of EEG electrodes to be standardized for further analysis. The 10-10 system of FIG. 1 is derived by modifying the conventional 10-20 system (as shown in FIG. 2) and is referred to as Modified Combinatorial Nomenclature (MCN). As illustrated in FIG. 2, the conventional system divides the scalp into 10% or 20% of the total front-back or right-left distance of the skull, and therefore the electrode spacing is proportional to the skull. EEG electrodes are placed to cover all the brain regions which are broadly categorized as Frontal (F), Temporal (T), Parietal (P), and Occipital (O). The numbering system is odd numbers to the left side and even numbers to the right side, while z represents midline numbers. The 10-10 system shown in FIG. 1 uses 1, 3, 5, 7, 9 for the left hemisphere which represents 10%, 20%, 30%, 40%, 50% of the inion-to-nasion distance, respectively. Compared to the 10-20 system of FIG. 2, in the 10-10 system of FIG. 1, the new letter codes are applied. The introduction of extra letter codes allows the naming of intermediate electrode sites, namely: AF - between Fp and F, FC - between F and C, FT - between F and T, CP - between C and P, TP - between T and P, and PO - between P and O, Also, the 10-10 system renames four electrodes of the 10-20 system: T3 becomes T7, T4 becomes T8, T5 becomes P7, and T6 becomes P8.

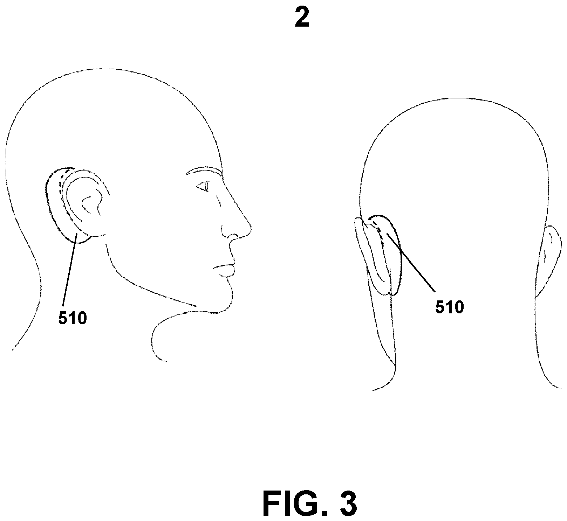

FIG. 3 is a diagram that illustrates a bony region behind the ears and can be used for the placement of reference electrode(s) according to various embodiments of the present invention.

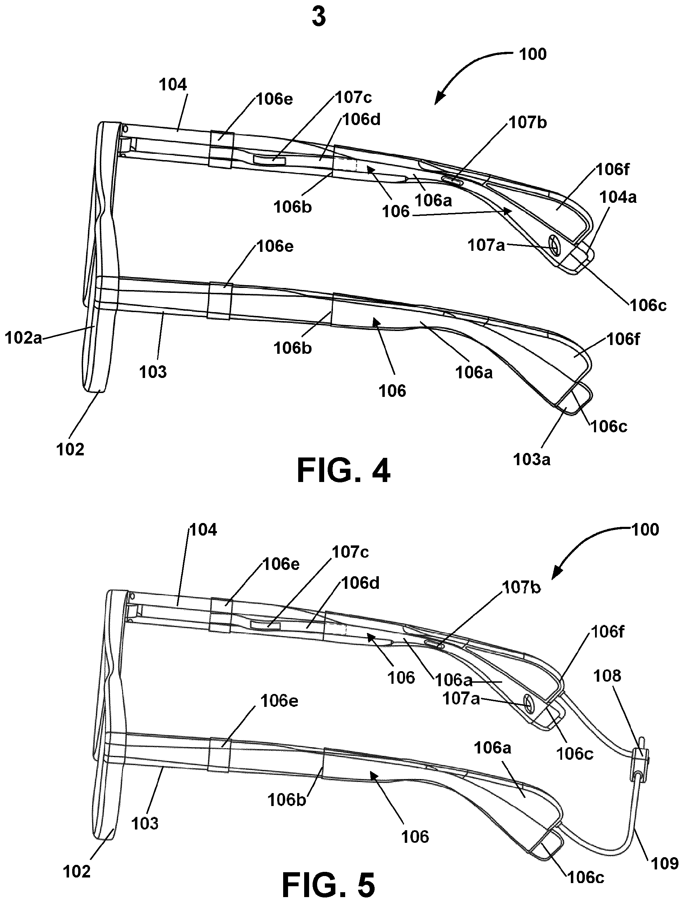

FIG. 4 shows an EEG device of an eyewear adapter mounted on eyewear, according to an embodiment of the present invention. As seen, the eyewear 100 like any conventional eyewear or glasses include a frame structure 102 for holding the eye lenses 102a, a left arm 103, and a right arm 104 where both arms are commonly referred to as temples, configured to rest over the user's ears when wearing eyewear 100. The two temples 103, 104 of the eyewear 100 are normally connected to the frame 102 at one end by hinges. These two temples also include temple tips/earpieces 103a, 104a at the other end.

The eyewear adapter 106 of the present invention includes a main structure 106a configured in the form of a sleeve so that the intended temples of the eyewear 100 (i.e. the left temple 103 or right temple 104) can be inserted by sliding inside the main structure of the adapter 106a. The main structure 106a may include one open aperture/opening or two open apertures/openings 106b, 106c at both ends as shown. In another embodiment, the main structure 106a may just include an opening or aperture at one end and be closed at the other end. In such case, only the opening 106b is present and the temple tips 103a, 104a of the eyewear 100 may not be visible but covered by the main structure 106a. Furthermore, the main structure 106a is preferably configured in a length of less than half of the temple 103, 104 of the eyewear 100. However, this should not be construed as a limitation. Additionally, the main structure 106a is made of elastic materials including but not limited to silicone, Thermoplastic Polyurethane (TPU), nitrile rubber, and other synthetic rubbers and has an adequate width of the openings so that every eyewear 100 can easily adapt to this main structure 106a by sliding it over the temples 103, 104. At the same time, this combination of material properties and the adequate width of opening also assures a tight fit and the fixed position of the main structure 106a over eyewear 100 during use.

The eyewear adapter 106 further includes a slider 106e, in forms of ring, clasp, or connector, which is made of similar material as that of the main structure 106a. The ring, clasp, or connector 106e is sized to fit over the temple of the eyewear 100 and can be slid over the temple 103, 104 of the eyewear 100 along the hinged end of the temple 103, 104 and the main structure 106a. In some other embodiment, the ring, clasp, or connector 106e may be configured in the form of a semicircular hook instead of a circular ring.

The eyewear adapter 106 further includes a strip 106d which is configured to connect the main structure 106a and the ring 106e. The strip 106d further embodies an EEG electrode 107c. The width of the strip 106d is preferably 2 mm which makes the eyewear adapter inconspicuous or invisible during use. The combination of the slider 106e and the strip 106d allows the user to place the EEG electrode 107c accurately at the locations FT9/FT10 of the 10-10 system (see FIG. 1, the temple area of the user's skull) by simply sliding the slider 106e toward either the hinged end of the temple 103, 104 or the main structure 106a using the fingers. Besides, the combination of the slider 106e and the strip 106d, and the elongated shape of EEG electrode 107c also allows the adjustment of the protruding structure of the stripe to optimize the contact pressure between the skin and the electrode 107c. This location of the electrode 107c on FT9/FT10 can also acquire the EOG signal that can be used for further interpretations such as facial expression of emotion recognition. The strip 106d may be made of a material including but not limited to Polyimide (PI) and Polyethylene terephthalate (PET).

In addition to the EEG electrode 107c, the main structure 106a embodies an EEG electrode 107a that is located at the rear part of the eyewear adapter 106, for example near the temple tip 103a, 104a of the eyewear 100 as shown in FIG. 4. In another embodiment, the EEG electrode 107c is located on the housing 106f. The EEG electrode 107a may be positioned in the vicinity of the bony region behind the user's ears as shown in FIG. 3 such as the positions A1 and A2 (mastoid points in FIG. 1). The main structure 106a embodies another EEG electrode 107b accurately positioned at T9/T10 position of the 10-10 system (as seen in FIG. 1). Besides, the adapter 106 may equip additional electrode(s) for EEG or EOG signal acquisition, e.g. an electrode as the bias input of the processing unit. The EEG eyewear adapter 106 of the present invention uses dry electrodes which are made of materials including but not limited to conductive polymer, non-skin-irritating metal, conductive ink, and conductive hydrogel. This application of EEG dry electrodes, requiring no extra preparation, facilitates the use of EEG measurement for all users.

The main structure 106a may comprise a flexible printed circuit (FPC). The strip 106d may also comprise an FPC. Single continuous FPC may comprise both the main structure 106a and the strip 106d. A FPC has a form which is relatively thin in the transverse thickness direction compared to the length and width and may be resilient to bending in the transverse direction. A FPC is arranged along the eyewear temples so that the thickness direction of the FPC is transverse to the eyewear temples and restoring force from the eyewear temples against the user's skull may be provided by the FPC. The EEG electrodes 107a, 107b, 107c are attached on the surface of the FPC, which works as an electrical connection. The restoring force of FPC urges the EEG sensor electrodes into good electrical contact with the user's skull.

The main structure 106a of the eyewear adapter 106 further includes a portion or housing 106f that houses a processing unit (not seen) therein in a concealed manner. In some embodiments, the housing 106f may also be positioned at the lower end of the temple tip 103a, 104a, and embody the processing unit therein.

Referring to FIG. 5 shows an eyewear adapter device mounted on eyewear, according to another embodiment of the present invention. The embodiment shown in FIG. 5 is essentially identical to the embodiment of the eyewear adapter 106 shown in FIG. 4 and thus lots of identical components and parts of the eyewear adapter 106 are not repeatedly described here in order to keep the description more concise. The main difference that can be noted in FIG. 5 is the location of the processing unit 108, which instead of being located in a concealed manner inside the housing 106f (as in FIG. 4) is separately located outside. The processing unit 108 is electrically connected to EEG electrodes 107a-107c and optionally any other additional electrode(s) of the eyewear adapter 106 using an electrical cable 109. It should be understood by those skilled that the eyewear adapter 106 may be designed to have more EEG electrodes without limitation to three EEG electrodes.

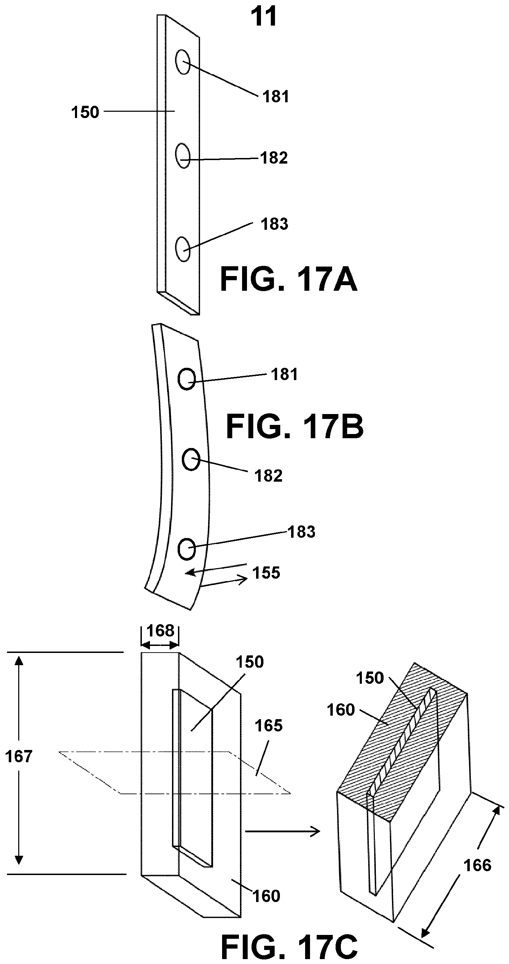

The EEG adapter (the main structure 106a, the slider 106e, the housing 106f, and so on) is made mainly of soft and elastic polymer(s) including but not limited to silicone and TPU instead of rigid materials. The electrical connectivity between EEG electrodes may be carried out by a flexible printed circuit (FPC) which allows direct electrode attachment, as seen in FIG. 17A where the exampled electrodes are annotated as 181, 182, and 183 on top of the FPC surface 150. This material organisation ensures a minimum thickness and dimension of the eyewear adapter 106. It can be pre-formed and restore the pre-formed shape against the applied force by its restoring force, as shown in FIG 17B the doubled arrows, which enables EEG electrodes in contact with the user's skull.

Depending on the electrode types, the manufacturing method of electrode attachment may vary. For example, when using conductive polymers as the electrodes, the electrode attachment may take place during the molding process. By applying pressure and heat, the polymer forms directly on the FPC which ensures strong and robust bonding. When using metallic electrodes, the metallic electrode may be either directly soldered onto the FPC or soldered after applying a solderable coating. As for electrodes made of conductive hydrogel or biomimetic material, the electrode attachment may involve additional electrode holders to host the electrodes mechanically such as with a structure of "teeth","tray", or "button", during or after the electrode being processed. Finally, for electrode materials with lower surface energies, the application of conductive adhesive at the interface may achieve the goal of a stabilized electrode attachment.

The FPC also works as the framework of the adapter 106 to maintain the overall shape. During the manufacturing process of the proposed EEG eyewear adapter, the FPC with the electrode(s) attached will be overmolded (using injection molding, compression molding, or similar molding processes) using the elastic polymer(s) as shown FIG. 17C where FPC 150 is embedded within an elastic material 160. For the proposed invention, the FPC is overmolded by one or more elastic materials on the area where no electrode is attached. The area with electrode attached, the electrode remains visible after overmolding, facilitating the skin contact for signal acquisition.

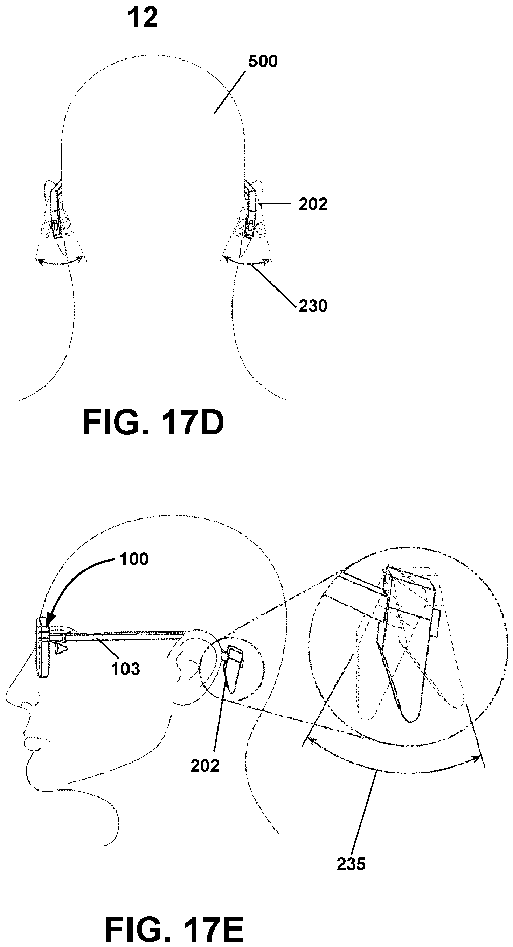

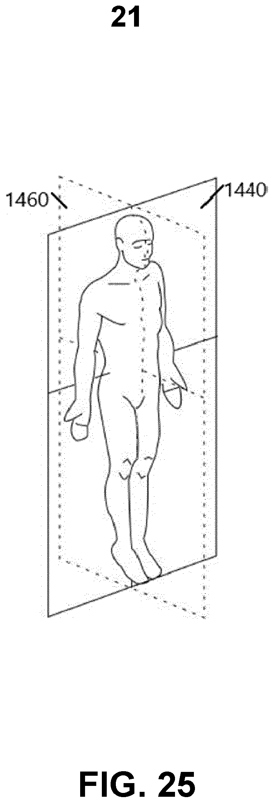

FPCs are in shape of a sheet with typical thicknesses of 0.13 mm to 0.2 mm versus lengths and widths normally at least over 1-2 mm. Due to the physical properties of FPC, i.e. in the form of a sheet and with high restoring force (as seen in FIG. 17B), after overmolding, the FPC permits movement and flexibility to the eyewear adapter 106 exclusively in the direction perpendicular to the FPC's sheetlike surface plane as illustrated by FIG. 17D. The FPC, however, forbids movement in the direction parallel to the median sagittal plane 235 as shown in FIG. 17E. The FPC in sheetlike or strip-like form is in a plane parallel to the median sagittal plane 1460 shown in FIG. 25.

In the eyewear adapter 106, the FPC's sheetlike surface plane faces toward the user's head, and therefore its movement toward or against the user's head is allowed, while the movement parallel to the median sagittal plane of the user is prohibited. With these physical properties of FPC, the adapter 106 can be adjusted for better contact of the EEG electrode 107a located in the movable regions at the rear part of the eyewear adapter 106 such as the housing 106f, in terms of signal quality and comfort by adapting to the head size of the user. This design ensures that the EEG electrode(s), including but not limited to 107a, at the bony region behind the ears especially at the mastoid positions A1/A2 in FIG. 1 can be well in contact to obtain the most desirable EEG signal quality.

Furthermore, the high restoring force of FPC is also advantageous to assure an adequate contact pressure of the EEG electrode 107c on the temple of the skull at the positions of FT9/FT10 in the 10-10 system, with the protruding structure created by adjusting the slider 106e toward the main structure 106a. The material proposed for EEG eyewear adapter also enables regular cleaning simply by using water to sustain the hygiene without damaging the functionality of the adapter.

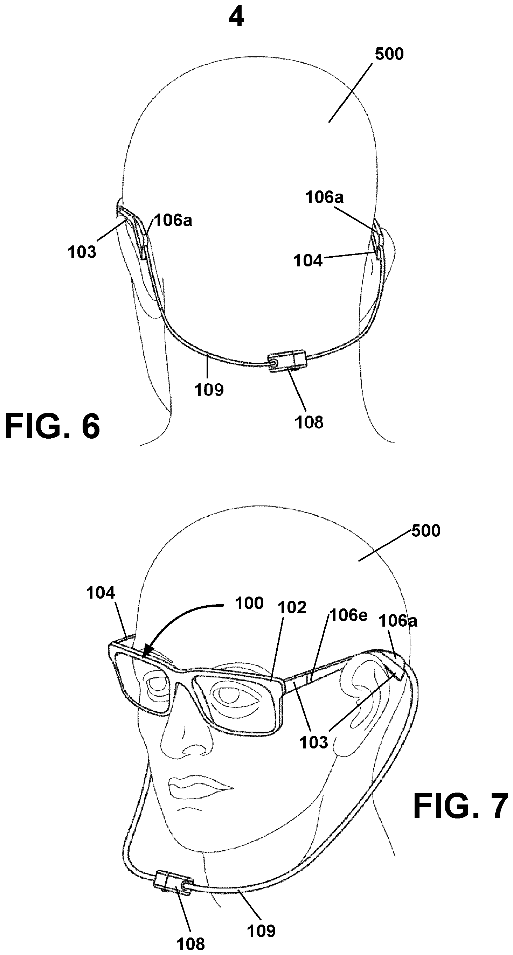

Referring to FIGS. 6 and 7, exemplary use case scenarios for the eyewear embodying the eyewear adapter of FIG. 5 are shown. As seen, the eyewear adapter 106 is mounted or worn over each of the two temples 103, 104 of the eyewear 100. When the eyewear 100 embodying the adapter 106 is worn by the user 500, the processing unit 108 may either be located behind the neck of the user (as shown in FIG. 6) or in front beneath the chin area as shown in FIG. 7. Depending upon the length of the electrical cable 109 used to connect the processing unit 108 and the adapter's EEG electrodes, the electrical cable 109 can work as a glasses cord when preferably positioned behind the neck of the user, and the processing unit 108 can also be aesthetically located in the form of a pendant in the front of the user 500 preferably near the user's chest region. Once the eyewear 100 with the adapter 106 is worn by the user 500, the electrodes 107b is positioned at T9/T10 because electrodes 107b is on the main structure proximate where it connects to the strip. The EEG electrode 107c is positioned on the strip 106d and can be adjusted by the user 500 by sliding the slider 106e. The contact of the EEG electrode 107a can be optimized by a tilt of the movable region such as the housing 106f carrying the EEG electrode 107a which is proximate the tip of the temple until the adapter 106 receives adequate EEG signals.

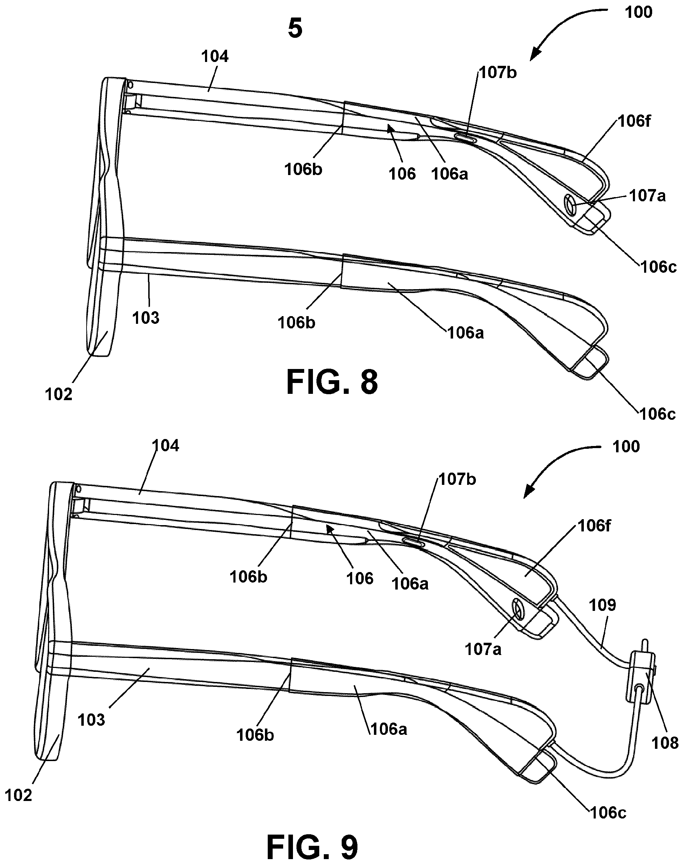

Referring to FIG. 8, an eyewear adapter device mounted on eyewear, according to another embodiment of the present invention is shown. The embodiment shown in FIG. 8 is essentially identical to the embodiment of the eyewear adapter 106 shown and described with respect to FIG. 4 and thus lots of identical components and parts of the eyewear adapter 106 are not repeatedly described here to keep the description more concise. The main difference that can be noted in FIG. 8 is the absence of the EEG electrode 107c, the slider 106e, and the strip 106d. This embodiment of the eyewear adapter 106 includes the EEG electrodes 107a-107b. The functionality of these electrodes 107a and 107b, the processing unit, and the configuration of the adapter's main structure 106a remain identical to the design described with respect to FIG. 4.

Referring to FIG. 9 shows an eyewear adapter device mounted on eyewear, according to another embodiment of the present invention. The embodiment shown in FIG. 9 is essentially identical to the embodiment of the eyewear adapter 106 shown and described with respect to FIG. 8 and thus lots of identical components and parts of the eyewear adapter 106 is not repeatedly described here to keep the description more concise. The main difference that can be noted in FIG. 9 with respect to FIG. 8 is the location of the processing unit 108, which instead of being located in a concealed manner is separately located outside. The processing unit 108 is electrically connected to EEG electrodes 107a, 107b, and optionally additional electrode(s) of the adapter 106 using the electrical cable 109.

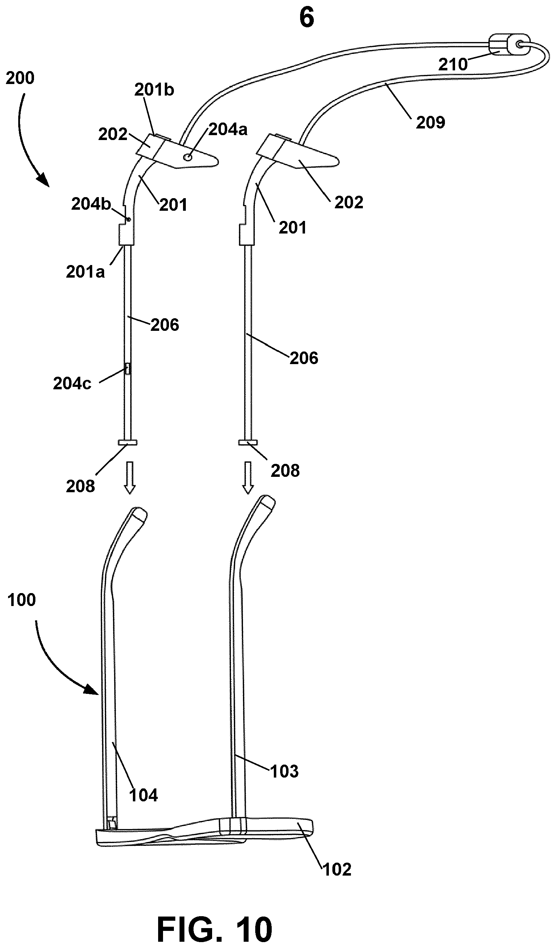

Referring to FIG. 10, an exploded view shows eyewear and an eyewear adapter according to another embodiment of the present invention. The eyewear adapter 200 is preferably made using the same constructional technique described above with respect to FIG. 17. In this embodiment, the eyewear adapter 200 includes a main structure 201 similar to the main structure 106a of FIGS. 4 and 5, the main structure 201 acts as a sleeve for the eyewear temples 103, 104 by sliding through the openings of 201a and 201b, where 201b is positioned before the temple tip of 103, 104.

As shown in FIG. 10 the main structure 201 has specifically a FPC embedded which acts as a substrate for the attachment of EEG electrodes. The EEG electrode 204b is similar to the EEG electrode 107b measuring EEG signals at the positions T9/T10 of the 10-10 system (see FIG. 1). At one end of the main structure 201 close to the temple tip of 103, 104, a curved earpiece 202 is located and is configured as an extension extending out of the main structure 201. This curved earpiece 202 is flexible and its flexibility comes from the combination of the internal FPC and the overmolding of the elastic polymer(s). This design organization ensures movement of the curved earpiece 202 in the direction toward or against the user's head (i.e. in the frontal plane of the user) and prevents movement of the curved earpiece 202 in the direction parallel to the sagittal plane of the user, ensuring the positioning of the EEG electrode 204a (located at the curved earpiece 202), the same positions as the EEG electrode 107a in FIGS. 4-5, in the vicinity of the bony region (indicated 501 in FIG3) when the eyewear adapter 200 is in use. Further, as seen, the eyewear adapter 200 includes a strip 206 that embodies an EEG electrode 204c and connects to the main structure 201 at one end. The strip 206 is operationally constructed and positioned similar to the strip 106d described above. The EEG electrode 204c measures EEG signals at the positions of FT9/FT10 of the 10-10 system (see FIG. 1) as 107c in FIGS. 4-5. The eyewear adapter 200 also includes a slider 208 operationally constructed and positioned similar to the slider 106e described above with reference to FIG. 4. The eyewear adapter 200 (a pair in fact, one for the left and one for the right side) fits over the temples 103, 104 of the eyewear 100 when in use. Additionally, just like the processing unit 108 shown in FIG. 5, a processing unit 210 in this embodiment is located outside of the main structure 201 and electrically connected to the EEG electrodes 204a-204c using an electrical cable 209.

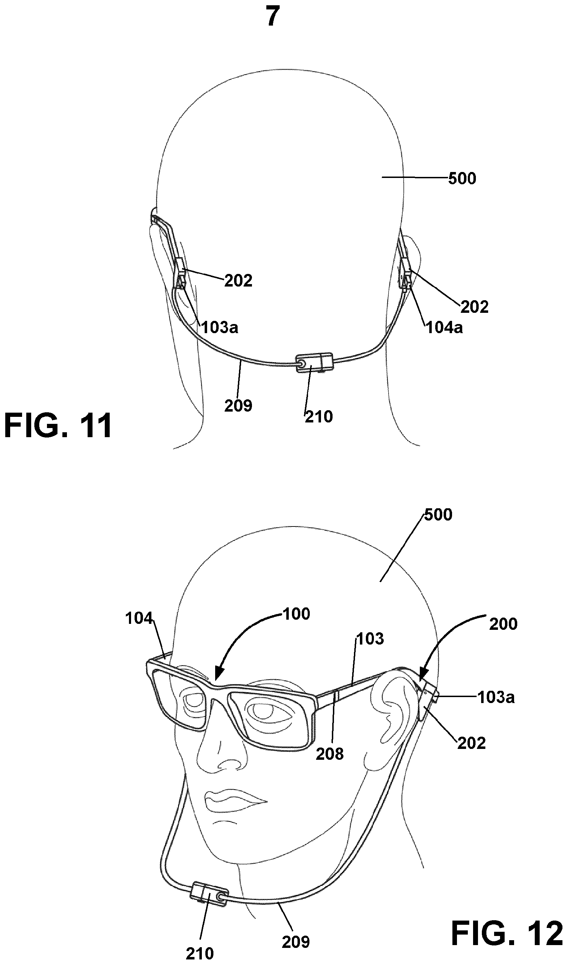

Referring to FIGS. 11 and 12, exemplary use case scenarios for the eyewear embodying the eyewear adapter of FIG. 10 are shown. As seen, the eyewear adapter 200 is mounted or worn over each of the two temples 103, 104 of the eyewear 100. When the eyewear 100 embodying the eyewear adapter 200 is worn by the user 500, the processing unit 210 can either be located behind the neck of the user (as shown in FIG. 11) or in front beneath the chin area as shown in FIG. 12. Depending upon the length of the electrical cable 209 used to connect the processing unit 210 and the adapter's EEG electrodes 204a-204c, the electrical cable 109 can work as a glasses cord when preferably positioned behind the neck of the user, and the processing unit 210 can also be aesthetically located in the form of a pendant when the combination of the processing unit 210 and the electrical cable 209 is located in front of the user 500 preferably near the user's chest region. Once the eyewear 100 with the eyewear adapter 200 is worn by the user 500, the positions of the electrodes 204a and 204c are adjustable whereas the position for the EEG electrode 204b is pretty much fixed. The position of the electrode 204a can be adjusted in the direction toward or away from the user's head by the user 500 to be located in the vicinity of the bony region (see FIG. 3) by adapting to the head size of the user with the flexible design of the curved earpiece 202. The adjustability of the EEG electrode 204c by the user 500 is done by sliding the slider 208 until the eyewear adapter 200 receives adequate EEG signals on the location FT9/FT10.

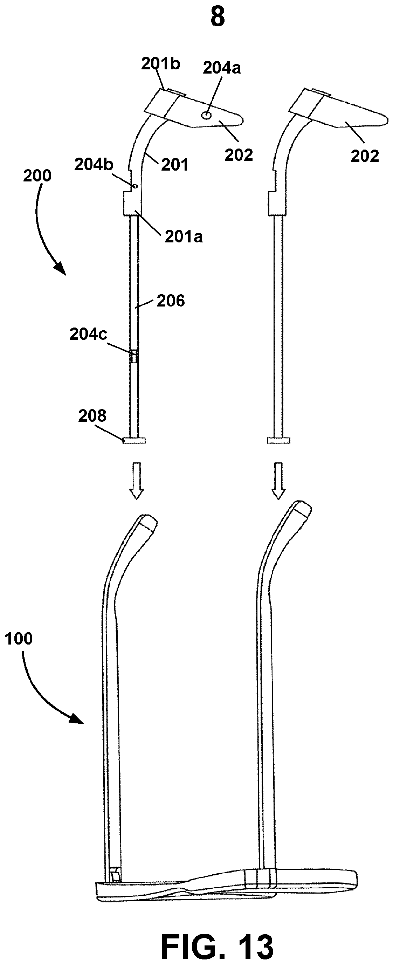

Referring to FIG. 13, an exploded view showing eyewear and an eyewear adapter according to yet another embodiment of the present invention. The eyewear adapter 200 shown in this embodiment is identical to the eyewear adapter described above with reference to FIG. 10. In contrast to the embodiment shown in FIG. 10, the design shown in FIG. 13 embodies the processing unit (not seen) within the main structure 201 or the curved earpiece 202 ensuring the processing unit remains concealed.

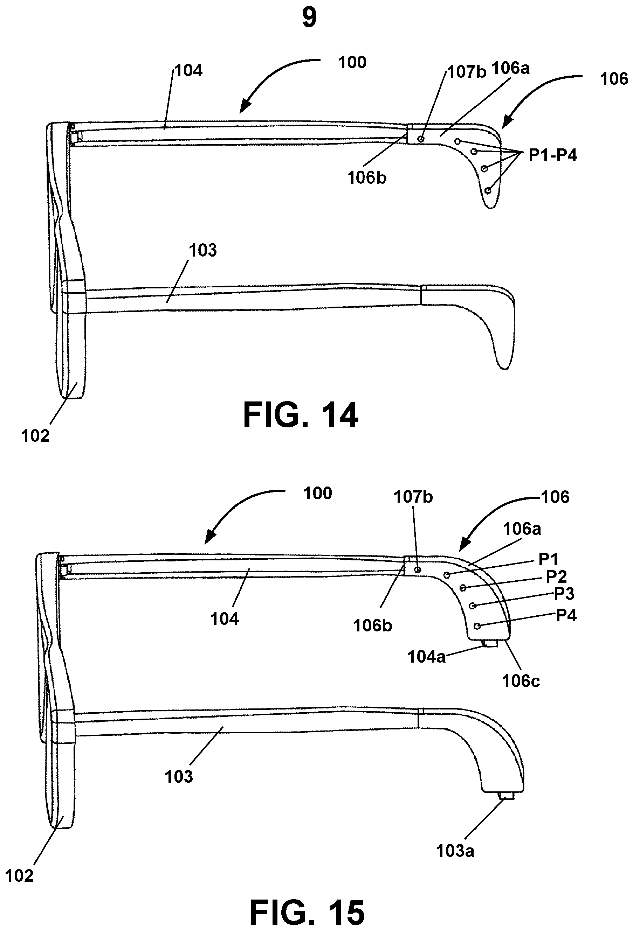

Referring to FIGS. 14 and 15, an eyewear adapter device mounted on eyewear, according to other embodiments of the present invention are shown. FIG. 14 shows an eyewear adapter 106 mounted over the temples 103, 104 of the eyewear 100. The adapter 106 fits over the temples 103, 104 and is oriented downward along with the temple tips of the eyewear 100. The adapter 106 acts as a sleeve with one open end 106b. The temples 103, 104 of the eyewear 100 are inserted inside the adapter 106 through the opening 106b. An EEG electrode 107b is accurately positioned at T9/T10 positions (as located in FIG. 1). Another EEG electrode(s) may be positioned within the downward extending portion of the eyewear adapter 106 that covers the bony region behind the user's ears (as shown in FIG. 3) when the eyewear adapter 106 is worn by the user. There could be multiple points/positions for placement of the electrode including but not limited to P1, P2, P3, and P4. The eyewear adapter shown in FIG. 15 is similar to the embodiment shown in FIG. 14; however, the adapter 106 of FIG. 15 is designed in the form of a sleeve with two open ends 106b, 106c. The adapter 106 design is such that it extends downward behind or before the temple tips 103a, 104a of the eyewear 100. The adapter 106 includes an EEG electrode 107b accurately positioned at T9/T10 positions (as located in FIG. 1). Another EEG electrode(s) may be positioned in an adapter portion that covers the bony region behind the ears of the user when the eyewear adapter 106 is worn by the user. There could be multiple points/positions for placement of the electrode(s) including but not limited to P1, P2, P3, and P4. The eyewear adapter 106 of FIGS. 14-15 may be formed using the same constructional technique as described above with reference to FIG. 17 to offer flexibility, ensuring optimized comfort and EEG signal quality. Although FIGS. 14-15 explicitly show the eyewear adapter 106 with one electrode 107b at T9/T10 and another electrode(s) that cover(s) the bony region behind the ears of the user (located at P1-P4 positions) which can be equivalent to the EEG electrode 107a as in FIGS. 4-5, it should be understood this embodiment can also be implemented for three electrodes on each side by adding one adjustable electrode positioned at FT9/FT10 using the setup of the slider 106e, the strip 106d, and the EEG electrode 107c described above in FIGS. 4-5. The processing unit is concealed inside the main structure 106a for both embodiments shown in FIGS. 14-15 and it should be understood that the processing unit can be placed outside the main structure 106a with the electrical cable as the combination of 108 and 109 shown in FIGS. 5, 9, 10.

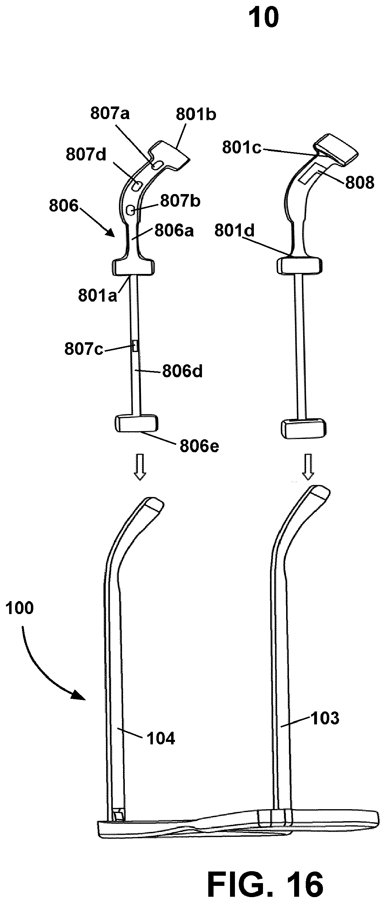

FIG. 16 shows an eyewear adapter 806 mounted over the temples 103, 104 of the eyewear 100. The adapter 806 fits over the temples 103, 104 and is oriented downward along with the temple tips of the eyewear 100. The adapter 806 is similar to the eyewear adapter 106, with the same structure of slidable setup to position the electrode 804c at the FT9/FT10 of the 10-10 system. The eyewear adapter 806 may have two open ends 801a and 801b to slide over the eyewear. The main structure 806a has a semi-tubular shape of sleeve comprising a major area faced inward to the user's skull and two open inner openings 801c and 801d forming the respective ring shape with the corresponding open ends 801b and 801a to grip the intended temple. With this semi-tubular shape of the main structure 806a, the intended temple tip remains visible when the eyewear adapter 806 is applied. The main structure 806a may host two electrodes 807a and 807b which corresponding to T9/T10 and the bony region such as A1/A2. One or more extra electrodes such as 807d may exist on the eyewear adapter 806 to stabilize the signal acquisition, i.e. working as the bias for electrical input. In this type of embodiments, the FPC is double-sided so that the soldering site or the electronic housing 808 may be on the opposite side of the main structure 806a. This double-sized FPC can minimize the dimension of the eyewear adapter 806. For some embodiments, the soldering site or the electronic housing 808 may host the entire processing unit and in some other embodiments, 808 only acts as soldering site to connect the external processing unit. The soldering site or the electronic housing may be covered by a protection cover, made of for example but not limited to polycarbonate (PC) or acrylonitrile butadiene styrene (ABS). In the case of having an external processing unit, the eyewear adapter 806 includes an electrical connection connecting the main structure and the processing unit.

It should be understood that the adapter's parts or components may be configured over both the temples of the eyewear.

Similar to the eyewear adapter, the proposed EEG monitoring device of an earwear facilitates EEG measurement for users or patients, especially for those who do not wear eyewear in daily life. The light, slim, and flexible design allows EEG measurements to be conducted invisibly and inconspicuously to capture the brain electricity of users and patient in real life for further interpretation from medical professionals.

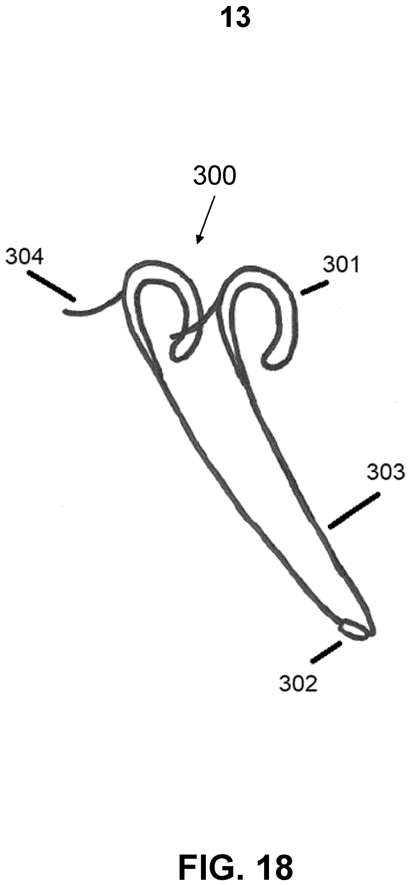

As shown in FIGS. 18 to 22 the invention may comprise earwear to be worn on or around a user's ears so that the EEG electrodes are placed in the appropriate places on the user's head.

The earwear may comprise a flexible printed circuit (FPC) which contains one or more EEG electrodes and a flexible material overmolded on top of the FPC. The flexible printed circuits may be connected to an EEG signal processing unit. As in the eye wear, in the earwear the FPC may provide the electrical connectivity between the EEG electrodes. The FPC allows direct EEG electrode attachment to ensure a minimum thickness/dimension of the earwear. The FPC may be made of a material including but not limited to polyimide (PI) and polyethylene terephthalate (PET).

FIG. 18 illustrates an embodiment of the invention that comprises one or more ear hooks 301. Each ear hook may comprise a FPC which contains one or more EEG electrodes and a flexible material overmolded on top of the FPC. The flexible printed circuits may be connected to an EEG signal processing unit 302 by an electrical wire 303. In other embodiments of the invention, the EEG signal processing unit may be directly concealed in one or more ear hooks 301 with the flexible printed circuits directly connected to the EEG signal processing unit. The FPC comprises resilient material which provides a restoring force to hold the EEG electrodes against the user's skull.

Some embodiments of the invention comprise a flexible extension 304 that extends from the ear hook 301. The flexible extension may be a part of the FPC of the ear hooks 301 and may also comprise EEG electrodes and a flexible material overmolded on top of the FPC. The purpose of the flexible extensions is to house EEG electrodes that contact the FT9/FT10 points of the user's head. Said FT9/FT10 points of the 10-10 EEG system are described further herein.

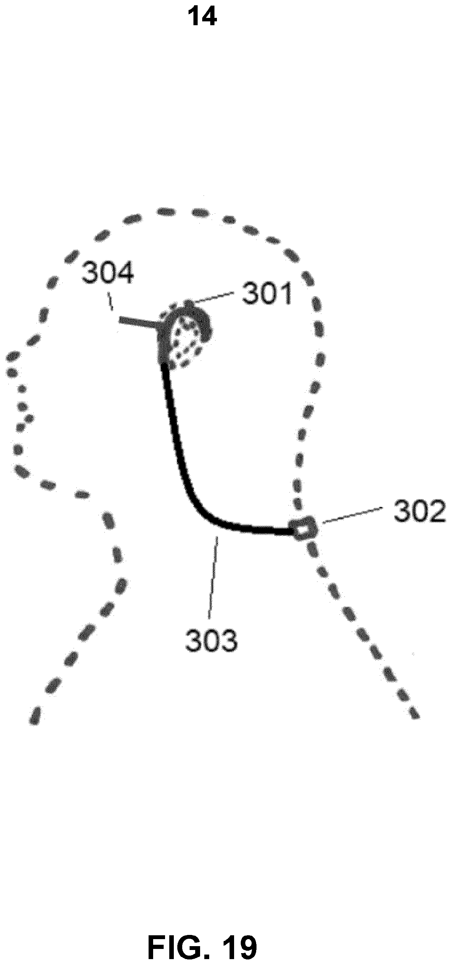

FIG. 19 illustrates the embodiment of the invention from FIG. 18 worn by a user. One of the one or more ear hooks 301, the EEG signal processing unit 302, at least a portion of the wire 303, and one flexible extension 304 are visible in FIG. 19. In the embodiment illustrated in FIG. 19, the EEG signal processing unit may rest below the user's neck when the invention is worn by a user. In other embodiments, the EEG signal processing unit may be located at any other point on a user's head, neck, or chest when the invention is worn by a user.

Also as illustrated in FIG. 19, the ear hooks may be worn around a user's outer ears between the helix of ear and the user's skull so that they contact the user's head and allow for proper placement of the EEG electrodes on the user's head. The FPC material of the ear hooks is flexible and adjustable in order to fit the user's head. In this manner, the invention is an improvement over the prior art in that it is easily size-adjustable by a user. Furthermore, skin adhesives may be applied on the embodiment, as the contour of the ear hook 301 and the extension 304, to fix the embodiment in place. The material of the skin adhesive may include but not limited to pressure sensitive adhesive (PSA) and biomimetic adhesive. Such an implementation of skin adhesive is able to ensure optimized signal acquisition from motion artifacts and coherent signal interpretation of EEG data. Said adhesives are described further herein.

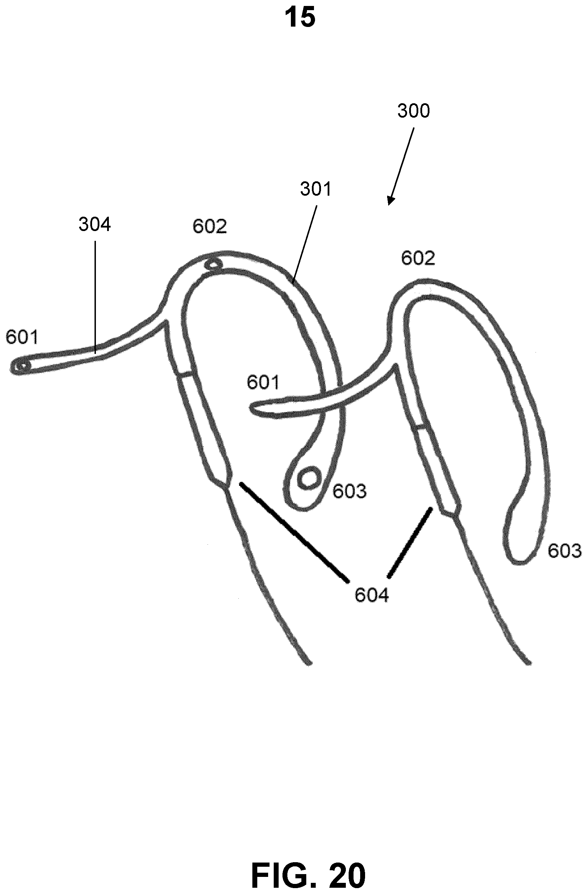

FIG. 20 illustrates a close-up view of the invention from FIG. 18. In FIG. 20, two ear hooks are visible with three EEG electrode locations designated by circles. The circles in FIG. 20 are only visible on one of the two illustrated ear hooks to show that the EEG electrodes in some embodiments of the invention are only visible on the side of the ear hooks that contact the user's head. Therefore, in the embodiments illustrated in FIG. 20, the ear hooks are illustrated at the angle in which only the EEG electrodes of one ear hook are visible.

The FPC in ear hooks is aligned so the plane of the sheetlike or strip-like form of the FPC is parallel to the plane of the ear hooks. When in use, the ear hooks are in a plane parallel to the median sagittal plane of the user. The FPC holds the EEG and/or EOG electrode(s). Movement parallel to the sagittal plane and the side of the user's head is resisted by the sheetlike or strip-like form of the FPC.

The EEG electrode locations illustrated in FIG. 20 are EEG electrode 601, EEG electrode 602, and EEG electrode 603. The EEG electrode locations may match up with specific EEG points of a user's head. Said points may be FT9/FT10 for EEG electrode 601, T9/T10 for EEG electrode 602, and A1/A2 for EEG electrode 603, according to the 10-10 system shown in FIG. 1. EEG electrode 601 may be located on the flexible extensions 304. Also visible in FIG. 20 are soldering positions 604 that connect the FPC to the electrical wire where the soldering are concealed inside the ear hook 301. In some embodiments of the invention, the soldering are also overmolded by the same flexible material that is overmolded onto the FPC. In some embodiments, the ear hook 301 may equip additional electrode(s) for EEG or EOG signal acquisition or as a bias input of the EEG signal processing unit.

During the manufacturing process of the proposed earwear, the earwear may also be manufactured including FPC. While or after the FPC is manufactured the EEC sensor electrodes may be attached to the FPC. the FPC with the EEG electrodes attached will be overmolded (using injection molding, compression molding, or similar molding processes) by an elastic material. FIG. 17C illustrates the configuration of materials in overmold where a material, in this case an FPC, is embedded within another material(s), here referring to an overmold material. This is to say that the overmold material will cover the main part of FPC and has openings at EEG electrode locations to allow the EEG electrodes to come in contact with the user's head. This is to say that in some embodiments, the EEG electrodes may come into account with a user's head, while the FPC is completely covered by the EEG electrodes and elastic polymer(s). The overmolded material may keep the physical properties of FPC, being resilient against deformation and reset to the pre-formed shape by the restoring force.

The overmold material comprises one or more flexible material such as a flexible plastic including but not limited to thermoplastic polyurethane (TPU), silicone rubber, nitrile rubber, and other synthetic rubbers. Said material is used to form the shape of ear hook. The FPC is overmolded within said material in order to provide the electrical connection and EEG electrode housing for the device. The resulting combination of FPC and overmold material is flexible and bendable especially transverse to its thinnest direction.

As in the eye wear, the application of FPC in the earwear assures EEG signal quality by providing flexibility and adjustability. An advantage of using an FPC, instead of electrical wires with attached EEG electrodes, is that the FPC/EEG electrode combination, i.e. direct attachment of EEG electrodes onto the FPC, is thin and flexible unlike traditional methods using rigid substrates carrying EEG electrodes. Therefore, the FPC/EEG electrode combination can adapt to different user' head sizes. For example the flexible extension 304 and/or ear hook or ear hook 301 shown in FIGS. 18 and 20 may comprise one or more FPC. The FPC may be embedded in an overmold material. The FPC comprises an EEG electrode 601, 602, 603.

Furthermore, the FPC has a high restoring force perpendicular to its sheet-like plane even after overmolding, which enables an adequate pressure from a pre-formed FPC applied on the EEG electrodes toward the skin. As shown in FIG. 17B, under an applied force, the FPC is able to restore to its original form after the release of force, annotated as the double arrows 155. The FPC permits flexibility to the device and movement exclusively in the direction perpendicular to the FPC's sheetlike surface plane. With these physical properties of FPC, each EEG electrode such as EEG electrode 601 in FIG. 20 on the extension of ear hook as described further herein can be adjusted for better contact of the EEG electrode therein onto the designated 10-10 positions, for example EEG electrode 601 to FT9/FT10, in terms of signal quality and comfort by adapting to the head size of the user.

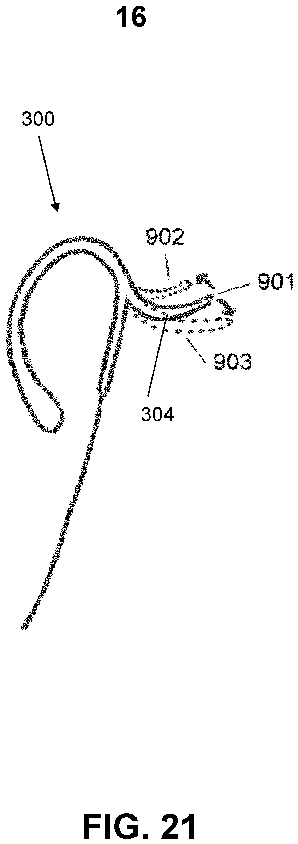

FIG. 21 illustrates the flexible extension (like extension 304 in FIG. 18) of the device in 3 positions, a first position 901 (on the surface plane/page), a second position 902 (into the plane/page), and a third position 903 (out of the plane/page). Arrows are illustrated to show the permissible movement of the flexible extension 304 between these 3 positions in the horizontal plane of the user.

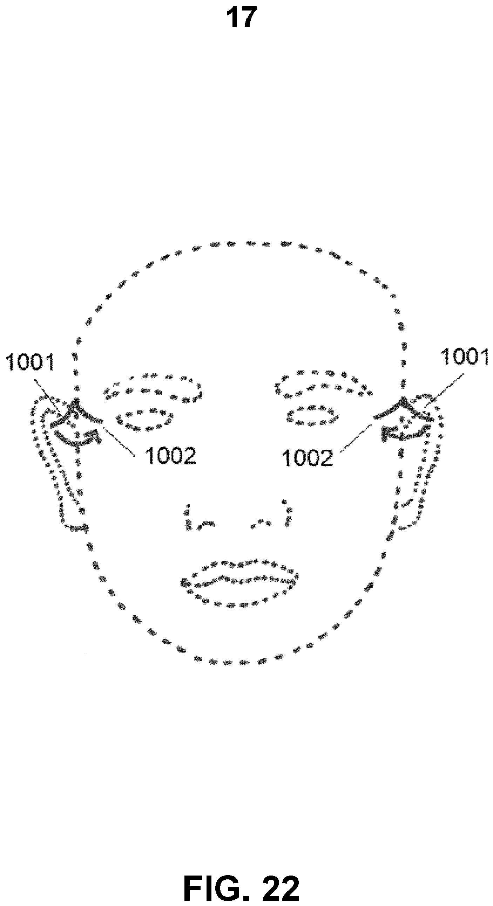

FIG. 22 illustrates a user's head that is wearing the EEG device. The flexible extensions 304 are visible in 2 positions, a first position 1001 and a second position 1002. As illustrated by the arrow showing the movement from the first position 1001 to the second position 1002, the flexible extension, an elastic material overmolding on a preformed FPC, is adjustable to adapt the size of user's head with an adequate pressure from the restoring force in order for the EEG electrodes located on the flexible extensions to come in contact with the user's head. Similarly, the FPC may also permit the EEG electrode 603 in FIG. 20 located on the ear hooks to be adjustable and fit on the individual's mastoid, according to the user's head size. In this way, the flexible nature of the FPC allows the device to be size-adjustable.

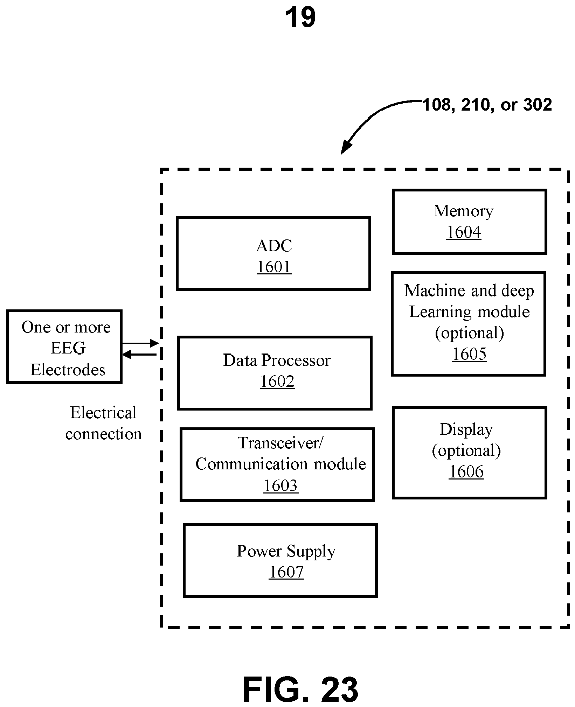

The processing unit preferably include an analog-to-digital converter (ADC) 1601, a data processor 1602, a transceiver/communication module 1603, a memory 1604, a machine learning and deep learning module 1605 embedded or stored in the memory 1604, a display 1606, and a power supply 1607 as shown in a general block diagram in FIG. 23.

In operation, the EEG electrodes 107a-107c, 204a-204c, P1-P4, 807c-807a, 807b, 807d configured on the eyewear adapter 106, 206, 806 acquire EEG signals from the user's brain through contact with the skull at any instant of time. Similarly EEG electrodes 601, 602, 603 on the ear hook 301 and extension 304 acquire EEG signals.

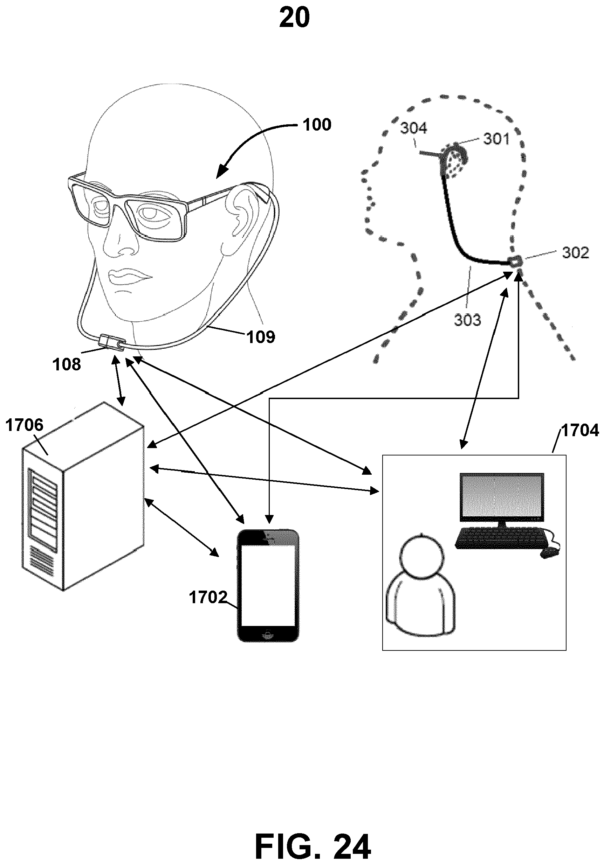

Once the EEG signals have been measured by one or more of the EEG electrodes of the proposed EEG monitoring devices, e.g. both the eyewear adapter and the earwear, the captured EEG signals in the analog form are transmitted to ADC 1601 of the processing unit. ADC 1601 converts analog EEG signals from the user to a digital form which is then communicated to the data processor 1602. The data processor 1602 includes one or more processors/micro-controllers known in the art and conducts signal analysis. This signal analysis by the processor 1602 is powered by machine learning or deep learning algorithms 1605 that may be optionally stored in the memory unit 1604 to distinguish if the captured signal by the EEG electrodes is within the acceptable EEG domain (e.g. frequency, amplitude). The machine learning or deep learning algorithms 1605 can be updated regularly via wired connection or via wireless communication. After the analysis is done by the processor 1602, the result is then displayed on the display unit 1606 if a display is present in the processing unit or transmitted via the transceiver/communication module 1603 to a user's device 1702 such as mobile phones or tablets for display as shown in FIG. 24. The EEG data from the processing unit is preferably transmitted using wireless technologies including but not limited to Wi-Fi and Bluetooth. The power supply 1607 is adapted to power the various components of the processing unit.

The pre-processed EEG data shown to the user/patient on one's mobile device 1702 reminds the user to adjust the EEG eyewear adapter 106 on the eyewear 100 properly if the acquired EEG signal quality is not ideal. Similarly for earwear, the user is prompted to adjust the ear hook and flexible extension 304. According to the embodiments of the present invention, the EEG data acquisition by the EEG electrodes will get initiated using either the user's mobile device 1702 or directly operating the processing unit of EEG eyewear adapter 106 or the earwear 300. Additionally, the user's mobile device may be enabled to generate corresponding EEG data in various formats (.csv, .mat, .xlxs, etc.) to facilitate communication with medical professionals. The EEG analysis can be processed inside the EEG eyewear adapter 106 or the earwear 300 (by the processor 1602 of the processing unit), the user's device 1702, or remotely on a computational cloud server 1706 via wireless communication. The server 1706 is preferably a high-end computing environment that includes all necessary components such as a storage unit, communication modules, processors, and so on. The technical details on the server 1706 are intentionally omitted in this disclosure as the server is well known in the art. The EEG data is then transferred to the mobile device 1702 to display the information to the user or the remote health facilities 1704, with the user's agreement for the purpose of patient monitoring, for further analysis or communication of health advice as shown in FIG. 24.

The invention has been described by way of examples only. Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the claims.

## Claims

1. An electroencephalographic (EEG) monitoring device comprising:
a main structure;
one or more flexible printed circuits (FPC) supporting the main structure;
one or more electroencephalographic electrodes on the flexible printed circuit(s);
an electroencephalographic processing unit electrically connected to the one or more flexible printed circuit(s),
wherein the EEG monitoring device is configured to contact one or more points on a user's head.

2. The EEG monitoring device according to claim 1 configured as an eyewear adapter device (106, 200, 800) for an eyewear (100), the main structure comprising at least one sleeve (106a, 201, 806) comprising the FPC, **characterised in that** the sleeve has at least one open end (106b, 201a, 801a) to slip over an intended temple (103, 104) of an eyewear and a distal end intended near the intended temple tip.

3. The EEG monitoring device according to claim 2 wherein the sleeve (106a, 201, 806) comprises at least one electrode positioned at T9/T10 and/or A1/A2 when in use.

4. The EEG monitoring device according to claim 1, 2 or 3 comprising one of the electrodes (107c, 204c, 807c) on a strip (106b, 206, 806d) of the FPC which connects the main structure to a slider (106e, 208, 806e) to force a portion of the strip (106b, 206, 806d), comprising the electrode (107c, 204c, 807c), to contact the position FT9/FT10 by moving the slider toward or away from the main structure.

5. The EEG monitoring device according to claim 4 wherein the strip is connected to the sleeve.

6. The EEG monitoring device according to claim 1 in a form of earwear (300) wherein the main structure comprises an ear hook comprising the FPC with least one of the electrodes intended to be positioned at T9/T10 and A1/A2, wherein the FPC has a sheetlike form coplanar with the ear hook.

7. The EEG monitoring device or adapter according to claim 6 comprising a flexible extension (304) extending from the ear hook (301), wherein the flexible extension comprises the FPC including at least one of the EEG electrodes positioned at FT9/FT10 of the 10-10 system.

8. The EEG monitoring device or adapter according to claim 6 or 7 in a form of earwear (300) comprising an adhesive to stabilize the contact with the user's scalp.

9. The EEG monitoring device or adapter according to claim 8 in a form of earwear (300) wherein the adhesive comprises a pressure sensitive material and/or a biomimetic material,

10. The EEG monitoring device (106, 206, 300, 806) according to any preceding clam wherein at least one of the EEG electrodes is a dry electrode comprising a conductive polymer, non-skin-irritating metal, conductive ink, or conductive hydrogel.

11. The EEG monitoring device (106, 206, 300, 806) according to any preceding claim wherein one or more soft and/or elastic polymers are applied to overmold or encapsulate the flexible printed circuit (FPC) to provide a selected flexibility and size-adjustability of the main structure.

12. The EEG monitoring device (106, 206, 300, 806) according to any preceding claim wherein the FPC, comprises a polyimide (PI) and/or polyethylene terephthalate (PET) material.

13. The EEG monitoring device of claim 1 comprising one or more electrooculographic (EOG) electrodes for analyzing information such as facial expression.

14. The EEG monitoring device according to any preceding claim configured symmetrically comprising a pair of the main structures, a pair of the FPC's, a pair of the EEG electrodes, for both sides of the user's head.

15. An eyewear or an earwear comprising an EEG monitoring device according to any preceding claim.
